# EUROPEAN PATENT APPLICATION

(11) **EP 2 248 482 A1**
(43) Date of publication of application: **10.11.2010**
(21) Application number: 10250881.9
(22) Date of filing: 05.05.2010
(51) Int. Cl.: A61B 19/00, A61B 17/34

(54) **Method for determining a position of an instrument within a body cavity**

(30) Priority: 06.05.2009 US 175912 P; 08.04.2010 US 756206
(71) Applicant: Tyco Healthcare Group LP, North Haven, CT 06473 (US)
(72) Inventor: Ransden, Jeffrey E., Fairfield CT 06825 (US); Helfer, Joel N., Cheshire CT 06410 (US)
(74) Representative: Soames, Candida Jane

(57) **Abstract**

A method for determining the depth, direction, and/or angle of an instrument inserted into a cavity in which a user compares the light output of a light source assembly at a distal end of the instrument with known light outputs for that light source assembly. The instrument may include a plurality of light source assemblies having light outputs of different colors and intensities. The instrument may be an introducer that enables a port, e.g., a foam port, to be accurately positioned within an incision.

## Description

### CROSS REFERENCE TO RELATED APPLICATION

The present application claims the benefit of and priority to U.S. Provisional Application Serial No. 61/175,912 filed on May 6, 2009, the entire contents of which are incorporated herein by reference.

### BACKGROUND

### Technical Field

The present disclosure relates generally to an access device for use in surgical procedures, and more particularly to a device and a method for determining the depth of an instrument within a body cavity.

### Background of Related Art

In several areas of surgery, it is frequently necessary to introduce tubular devices into the operative site. Typical of these areas of surgery is laparoscopic surgery, in which surgical instruments are inserted through the abdominal wall to reach an operative site within the operative site within the abdominal cavity.

For example, single incision laparoscopic surgical techniques typically involve delivering a port through an incision created at a desired location in a patient's abdomen. This is achieved by mounting a port to an introducer and advancing the introducer through the tissue layers within the abdomen. Once the introducer has entered the abdomen, the port is separated from the introducer and the introducer is removed from the incision.

Clinicians may have difficulty determining the extent of penetration of the introducer into the peritoneum. If clinicians were able to more accurately determine the position of the introducer, they would be able to achieve a better placement of the port while minimizing the risk of damage to adjacent body structures.

### SUMMARY

The present disclosure is directed to a method for determining the position of an instrument within a body cavity.

In an embodiment, a method of determining a position of an instrument is disclosed including inserting an instrument into a body cavity, the instrument including at least one light source at a distal end thereof, repositioning the instrument, observing an output of the at least one light source, and determining a position of the instrument by comparing the observed output of the at least one light source to a known output of the at least one light source. The light source may emit light at a constant rate of output. The at least one light source may also include a plurality of light sources of different colors or intensities. A template may further be provided such that a clinician may compare the observed light with known light outputs for the light sources at given depths. The instrument may also include a light sensor.

In an embodiment, the instrument is an introducer device. The method may also comprise the step of: prior to the inserting step, mounting a port to the introducer such that the introducer and the port are inserted into the body cavity together. In such an arrangement, the port may be a foam port. Also, the method may comprise the step of: after the determining step, removing the introducer from the body cavity while maintaining the port within the body cavity.

### BRIEF DESCRIPTION OF THE DRAWINGS

Embodiments of the disclosure will be described with reference to the accompanying drawings in which:

Fig. 1 is a view of an instrument in accordance with an embodiment of the present disclosure;

Fig. 2 is a view of the instrument within a body cavity; and

Fig. 3 is a view of an instrument in accordance with a further embodiment the present disclosure.

### DETAILED DESCRIPTION

Particular embodiments of the present disclosure will be described herein with reference to the accompanying drawings. In the following description, well-known functions or constructions are not described in detail to avoid obscuring the present disclosure in unnecessary detail.

As shown in the drawings and as described throughout the following descriptions, and as is traditional when referring to relative positioning on an object, the term "proximal" refers to the end of a device that is closer to the user and the term "distal" refers to the end of the device that is further from the user.

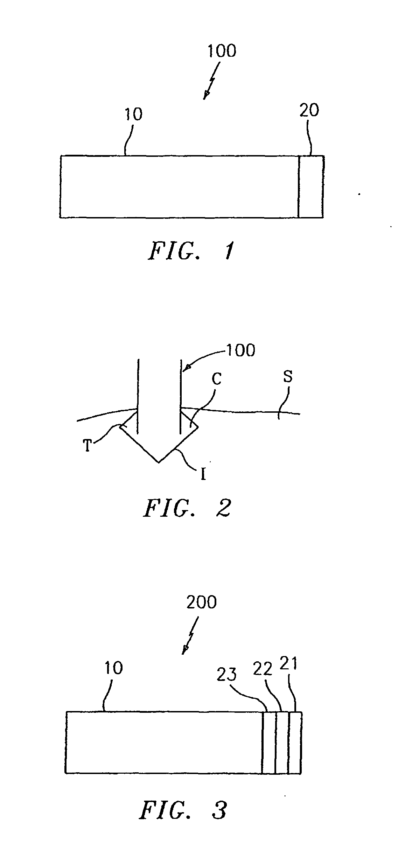

An instrument 100, as shown in Fig. 1, includes a shaft 10 having a light source assembly 20 at a distal end. The light source assembly 20 may include a plurality of light emitting diodes 22 mounted about an outer diameter of the shaft 10. As shown in Fig. 2, the instrument 100 may be inserted into an incision I within a patient's skin S. As the instrument 100 is inserted into the incision I by a clinician, a small ring of tissue T adjacent to the light source assembly 20 will be illuminated. By advancing the instrument 100 further into a body cavity C, a change in light as perceived by the clinician will be apparent. Instead of a small ring of tissue illuminated by the adjacent light source assembly 20, the illumination will be more profuse when in the body cavity C, i.e., the amount of light perceived by the clinician will be noticeably less.

By comparing the observed light output for each repositioning of the instrument 100 within the body cavity C against known light outputs for the light source assembly 20, the clinician may determine the depth, angle, and direction of insertion of the instrument 100.

In a further embodiment, shown in Fig. 3, an instrument 200 includes a shaft 10 and a plurality of light source assemblies 21, 22, and 23 mounted along a shaft 10. Each of the light source assemblies 21, 22, and 23 emit light of a different color or intensity. As the clinician inserts the instrument 200 deeper within the body cavity C, each of the light sources 21, 22, and 23 will become less visible. By observing which of the light source assemblies 21, 22, and 23 the clinician will be able to determine the position of the instrument 200 by comparing the observed light output to the known outputs for each of the light source assemblies and the known position of each of the light source assemblies along the shaft 10.

A template may be provided by which the clinician may compare the observed light output against the known light outputs for a given depth for a particular model light source assembly. The instrument 100, 200 may also include a light sensor (not shown) to record and display the observed light outputs such that the position of the instrument 100, 200 may be determined.

The instrument 100, 200 may also include an internal power source (not shown) and switches for completing a circuit (not shown) to energize the light source assemblies 20, 21, 22, and 23.

It will be understood that various modifications may be made to the embodiments disclosed herein. For example, the location, size, and type of light source used may be modified to better suit a particular surgical procedure. In addition, it is understood that the methods disclosed herein for determining a position of an instrument within a cavity may have applications in other fields than those discussed herein. Therefore, the above description should not be construed as limiting, but merely as exemplifications of various embodiments. Those skilled in the art will envision other modifications within the scope and spirit of the claims appended hereto.

## Claims

1. A method for determining a position of an instrument within a body cavity, comprising the steps of:
inserting an instrument into a body cavity, the instrument including at least one light source at a distal end thereof;
repositioning the instrument;
observing an output of the at least one light source; and
determining a position of the instrument by comparing the observed output of the at least one light source to a known output of the at least one light source.

2. The method of claim 1, wherein the at least one light source emits light at a constant rate of output.

3. The method of claim 1 or claim 2, wherein the at least one light source includes a plurality of light sources each emitting light of a different color or intensity.

4. The method of any preceding claim, wherein the instrument is an access device.

5. The method of any preceding claim, wherein a template is provided having the known light outputs at given positions within a body cavity.

6. The method of any preceding claim, wherein the instrument further comprises a light sensor.

7. The method of any preceding claim, wherein the instrument is an introducer device.

8. The method of claim 7, further comprising the step of:
prior to the inserting step, mounting a port to the introducer such that the introducer and the port are inserted into the body cavity together.

9. The method of claim 8, wherein the port is a foam port.

10. The method of claim 8 or claim 9, further comprising the step of:
after the determining step, removing the introducer from the body cavity while maintaining the port within the body cavity.
